# FASCICULE DE BREVET EUROPEEN

(11) **EP 0 813 063 B1**
(45) Date de publication et mention de la délivrance du brevet: **20.07.2005**
(21) Numéro de dépôt: 97420084.2
(22) Date de dépôt: 04.06.1997
(51) Int. Cl.: G01N 33/34, G01N 21/85, G01N 15/02, D21G 9/00, B01D 33/00

(54) **Procédé de régulation d'une chaîne de désencrage de pâte à papier et dispositif pour mesurer en continu la quantité de particules contenues dans un liquide**
Regelverfahren für eine Anlage zum Deinken einer Fasersuspension und Vorrichtung zur kontinuierlichen Messung der in einer Flüssigkeit befindlichen Teilchenmenge
Method of controlling equipment for deinking paper pulp and device for continuously measuring the quantity of particles in a liquid

(30) Priorité: 10.06.1996 FR 9607391
(43) Date de publication de la demande: 17.12.1997
(73) Titulaire: CENTRE TECHNIQUE DE L'INDUSTRIE DES PAPIERS CARTONS ET CELLULOSES ., 38100 Grenoble (FR)
(72) Inventeur: Eymin Petot Tourtollet, Guy, 38570 Tencin (FR); Julien Saint Amand, François, 38660 Le Touvet (FR); Perrin, Bernard, 38100 Grenoble (FR); Sabater, Jacques, 91190 Gif Sur Yvette (FR)
(74) Mandataire: Vuillermoz, Bruno

(56) Documents cités:
- EP-A- 0 059 140
- EP-A- 0 310 740
- WO-A-86/05525
- WO-A-95/08019
- FR-A- 2 297 413
- US-A- 4 911 025
- US-A- 5 191 388

## Description

L'invention concerne un procédé destiné à permettre la régulation d'une chaîne de désencrage d'une pâte à papier obtenue à partir de papiers recyclés ou de récupération.

Elle concerne également le dispositif susceptible de mettre en oeuvre ce procédé, et de manière plus générale, un dispositif susceptible de mesurer en continu la quantité de particules contenues dans un liquide, lesdites particules se présentant notamment sous deux états physiques différents, notamment à l'état individualisé, et à l'état lié à une entité de plus grande dimension, telle que par exemple une fibre, ces entités étant notamment microscopiques et étant préalablement séparées selon des critères de dimensions par des moyens mécaniques.

L'invention concerne plus particulièrement la séparation continue par tamisage et lavage entre des particules microscopiques individualisées dans une suspension et des particules microscopiques liées à d'autres entités plus volumineuses de ladite suspension, suivie d'une mesure en continu par analyse d'images sur les flux de suspension séparée des diverses fractions de particules.

Comme déjà précisé, l'invention est plus particulièrement destinée à l'industrie papetière, notamment, pour le contrôle et la régulation des opérations de désencrage des pâtes à papier issues des papiers de récupération, en procurant des moyens de mesure séparée des fractions de particules d'encre "accrochées" et "décrochées" des fibres papetières, notamment cellulosiques, et ce directement et en continu dans la pâte à différents niveaux du processus de désencrage.

Bien entendu, si dans la suite de la description, l'invention est plus particulièrement décrite dans son application à l'industrie papetière, elle ne saurait se limiter à cette seule application, et peut également être mise en oeuvre dans d'autres domaines nécessitant le contrôle continu et séparé de particules microscopiques en suspension, selon leur nature, lié à des critères de dimension, notamment dans le domaine du traitement des eaux, dans celui des industries chimiques ou agro-alimentaires.

Le recyclage des papiers de récupération nécessite, en particulier dans le cas des vieux papiers imprimés devant être recyclés dans la fabrication des papiers blancs, notamment de type graphique, la mise en oeuvre de diverses opérations de désencrage visant à éliminer les encres d'imprimerie associées à ces matières. L'élimination de ces particules d'encre implique tout d'abord qu'elles soient individualisées, c'est à dire correctement séparées du support d'impression papier, et en particulier qu'elles soient décrochées des fibres afin de pouvoir être extraites sélectivement de la suspension de fibres recyclées.

Le contrôle séparé et en continu des particules d'encre accrochées et décrochées s'avère tout à fait avantageux, dès lors que les opérations de décrochage de l'encre, notamment par pulpage et par trituration à chaud, et les opérations d'élimination de l'encre, notamment par lavage et par flottation, font appel à des technologies et à des adjuvants chimiques différents.

De manière traditionnelle, les procédés de désencrage comportent la plupart des étapes suivantes :
- mise en pâte des vieux papiers par pulpage, c'est à dire par action mécanique en présence de produits chimiques favorisant le défibrage des papiers et le décrochage de l'encre des fibres ;
- pré-épuration par classage, sur tamis à trous et à fentes, et par cyclonage, destinée à éliminer les impuretés volumineuses et lourdes ;
- désencrage par flottation et/ou lavage, visant à éliminer les particules d'encre dans la mesure où elles ont été séparées des fibres lors du pulpage ;
- épuration fine par classage à fentes et cyclonage pour éliminer des petites impuretés lourdes et légères ;
- traitement à chaud visant d'une part, à décrocher les particules d'encre restées fixées aux fibres et d'autre part, à disperser ou fragmenter les impuretés résiduelles en dessous du seuil de visibilité (fragments d'impression et impuretés diverses, notamment thermofusibles) ;
- blanchiment de la pâte, pouvant être combinée à la dispersion à chaud lorsque les produits de blanchiment sont introduits en amont du traitement à chaud.
Le document WO - A- 8605525 décrit un procédé de ce type, visant à mesurer les particules lourdes et légères.

D'autres étapes peuvent compléter ces opérations, en particulier lorsque les exigences de qualité de la pâte désencrée sont très élevées en termes de blancheur et de propreté. Ces étapes sont essentiellement constituées par :
- post-désencrage par flottation et/ou lavage, destiné à éliminer les encres ayant été décrochées lors de la dispersion à chaud ;
- post-épuration par classage à fentes fines et/ou cyclonage, permettant de parfaire l'épuration, notamment du fait des modifications de forme subies par des impuretés de type généralement désignées selon l'expression en langue anglaise "stickies" (colles et adhésifs) au cours du traitement à chaud ;
- traitement à chaud final permettant de disperser les encres et impuretés résiduelles en dessous du seuil de visibilité ;
- blanchiment complémentaire de type en principe différent, c'est à dire réducteur lorsque le premier blanchiment est oxydant, et inversement.

Les dimensions des particules d'encre dans les pâtes recyclées sont typiquement de l'ordre de quelques micromètres. La répartition de tailles de ces particules peut varier considérablement suivant la nature des encres, c'est à dire inclure des dimensions inférieures au micromètre pour certaines encres de type hydrosoluble (impression flexographique,...) ou dans le domaine du visible voire jusqu'à plusieurs centaines de micromètres, pour certaines encres du type thermofusible (encres laser,...).

Il existe à ce jour des possibilités de contrôle en continu directement dans la pâte des particules contrastées. Cependant, les techniques actuellement proposées se limitent à la mesure de particules de dimensions supérieures au seuil de visibilité. Un dispositif de contrôle de ce type de particules est par exemple décrit dans le document EP-B-0 539 456.

Un tel dispositif de contrôle comporte fondamentalement une canalisation de dérivation munie d'une fenêtre transparente au regard de laquelle est mise en place une source lumineuse impulsionnelle, destinée à éclairer ladite fenêtre, et une caméra destinée à détecter en rétrodiffusion les impuretés de contraste et à les mesurer, de telle sorte à extraire des signaux ainsi captés la surface apparente de chacune desdites impuretés et la quantité des impuretés. Ce dispositif comporte également des moyens de numérisation et de traitement afin de restituer une forme visuelle et exploitable de ces données.

Ces techniques de mesure en ligne des points noirs constituent des moyens de contrôle efficaces pour certains aspects de désencrage mais ne permettent pas néanmoins de contrôler les aspects relatifs au critère essentiel de blancheur de la pâte, à savoir l'élimination des encres microscopiques et leur décrochage préalable.

Les moyens de contrôle en ligne de ces aspects essentiels du désencrage se limitent actuellement à des mesures de blancheur de la pâte en milieu liquide par des techniques d'analyse de lumière rétrodiffusée.

Ces mesures de blancheur fournissent des indications sur les variations de qualité de la matière première et/ou d'efficacité des opérations de désencrage, mais ne permettent d'évaluer ni l'efficacité d'élimination de l'encre, ni a fortiori l'efficacité du décrochage, dans la mesure où la blancheur des pâtes est fortement affectée par leur composition en charges minérales et fibres de différentes origines.

Ainsi, une blancheur insuffisante en fin de chaîne de désencrage peut avoir des causes très diverses, comme par exemple une efficacité de flottation limitée, la présence d'encre difficile à décrocher, ou de mauvaises conditions de blanchiment, qui ne pourront être détectées par les mesures de blancheur en ligne.

Des techniques de laboratoire sont également utilisées pour l'évaluation du désencrage, mais elles ne permettent pas le contrôle en ligne et, nécessitent des procédures de prélèvement et de préparation d'échantillons ainsi que la mise en oeuvre de mesures plus ou moins longues et fastidieuses. Parmi celles-ci, on peut mentionner la mesure globale de teneur en encre obtenue par analyse de réflectance en lumière visible et proche infrarouge, et ce sur échantillons de pâte lavée ou épaissie sur filtre après adjonction de floculants, mais également la mesure directe des particules d'encre obtenue par analyse d'images sous microscope, et ce sur diverses préparations d'échantillons de pâte, de fibres lavées ou de filtrats déposés sur microfiltres.

L'objet de l'invention est de pallier ces inconvénients et de s'affranchir des limites des moyens actuels de contrôle en ligne du désencrage.

Elle concerne en particulier des moyens de séparation continue, par tamisage et lavage entre les particules d'encre accrochées et décrochées, en suspension dans les pâtes issues de papier de récupération, associés à des moyens de mesure continue des quantités et des dimensions de ces deux fractions de particules d'encre, et ceci par analyse d'image sur les flux des suspensions séparées.

De manière plus générale, elle concerne un dispositif pour mesurer en continu la quantité des particules se présentant sous des états physiques différents, en suspension dans un liquide. Ce dispositif comprend :
- un ensemble de séparation et de lavage des particules d'encre en fractions respectivement accrochées et décrochées, ledit ensemble étant alimenté en continu en pâte à papier par une canalisation appropriée, et comprenant :
   - un tamis micro-perforé cylindrique à l'intérieur duquel est acheminée en continu par une canalisation la pâte à papier à analyser ou
      un tamis micro-perforé plan au niveau duquel est acheminée en continu par une canalisation la pâte à papier à analyser ;
   - un organe de décolmatage et de classage destiné à assurer la création de pulsations de pression, et à entraîner la pâte à papier à la surface du tamis ;
   - des canalisations de collecte et de sortie, respectivement des particules d'encres décrochées non diluées, des éléments fins dilués, et des fibres papetières lavées avec particules d'encre accrochées ;
- deux canalisations ou veines, issues dudit organe de séparation, acheminant lesdites fractions au niveau d'une zone de mesure et comportant au niveau de cette zone une fenêtre transparente ;
- un ensemble de mesure et d'acquisition comprenant :
   - un organe d'éclairage desdites fenêtres ;
   - deux caméras situées en regard de chacune desdites fenêtres, synchronisées avec ledit organe d'éclairage et destinées à acquérir à intervalles réguliers une image des fenêtres ;
   - des moyens de numérisation et de traitement des images ainsi acquises, destinés à déterminer la dimension et la quantité desdites particules ;
   - un organe de sortie des données ainsi déterminées, notamment vers un organe de restitution sensorielle, ou sous la forme de signaux analogiques ou numériques, destinés à actionner un ou plusieurs organes déterminés.

Au sein de cet organe de séparation et de lavage comprenant un tamis cylindrique micro-perforé tourne de préférence de manière continue un rotor de décolmatage et de classage, dont l'axe de rotation est colinéaire avec l'axe du tamis, et assurant la création de pulsations de pression, et entraînant le liquide à la surface interne du tamis.

L'invention a pour objet également un procédé de régulation d'une chaîne de désencrage de pâte à papier issue de papiers recyclés, qui consiste à mesurer en continu la quantité des particules d'encre en suspension sous deux états physiques différents, respectivement individualisées au sein de la pâte et liées à d'autres particules plus volumineuses, et notamment aux fibres papetières, puis en fonction des mesures ainsi effectuées, à agir au niveau des différents paramètres de fonctionnement de la chaîne de désencrage, notamment lors des étapes de pulpage et de trituration à chaud, et lors des étapes de lavage et de flottation.

La manière dont l'invention peut être réalisée et les avantages qui en découlent, de même que ses applications ressortiront mieux des exemples de réalisation qui suivent donnés à titre indicatif et non limitatif à l'appui des figures annexées.
La figure 1 est une représentation schématique de l'installation conforme à l'invention.
La figure 2 est une représentation schématique en section transversale d'un détail de la figure 1, dont la figure 3 est une représentation en section longitudinale.

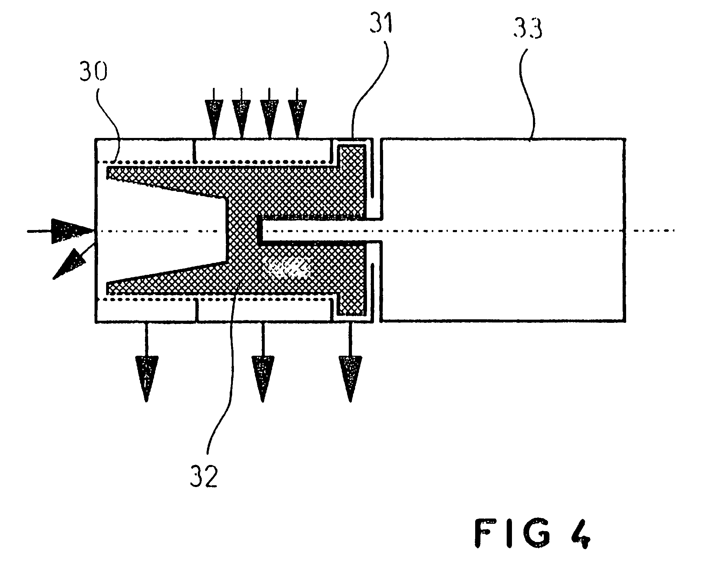

La figure 4 est une représentation schématique du dispositif de séparation et de lavage d'une pâte à papier conforme à l'invention, dont la figure 5 est une vue plus détaillée en section.
La figure 6 représente une autre forme de réalisation du dispositif des figures 4 et 5.

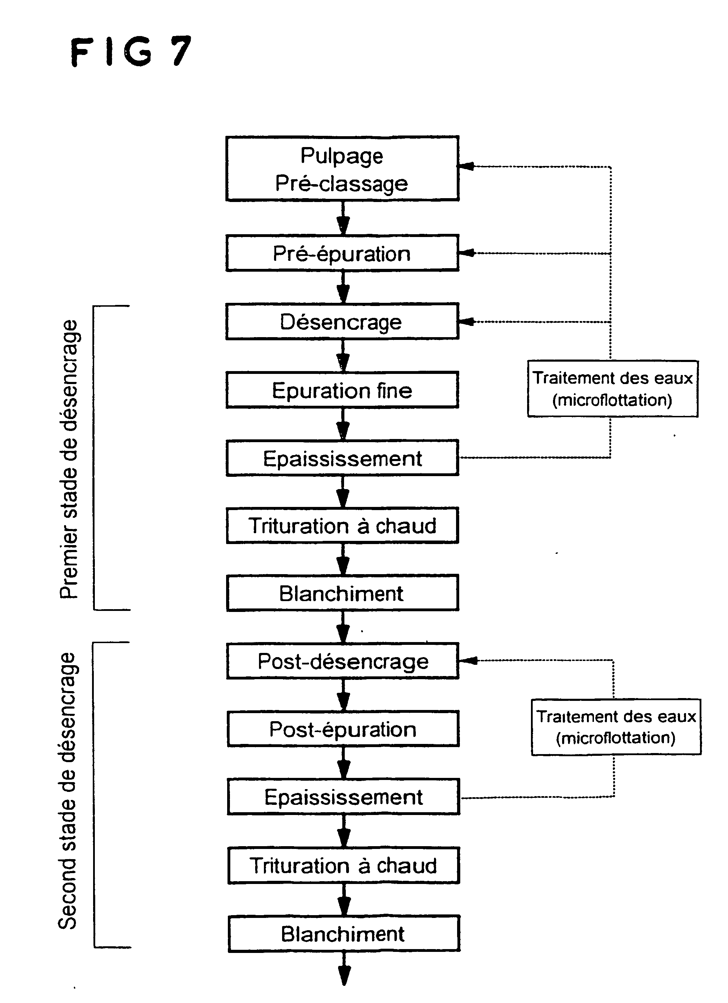

La figure 7 est une forme simplifiée d'un diagramme représentatif des différentes étapes de traitement au sein d'une chaîne de désencrage.

La description qui suit concerne une installation destinée à être montée au niveau d'une chaîne de désencrage de pâte à papier. Ainsi que déjà dit, il est bien entendu que l'invention ne saurait se limiter à cette seule application.

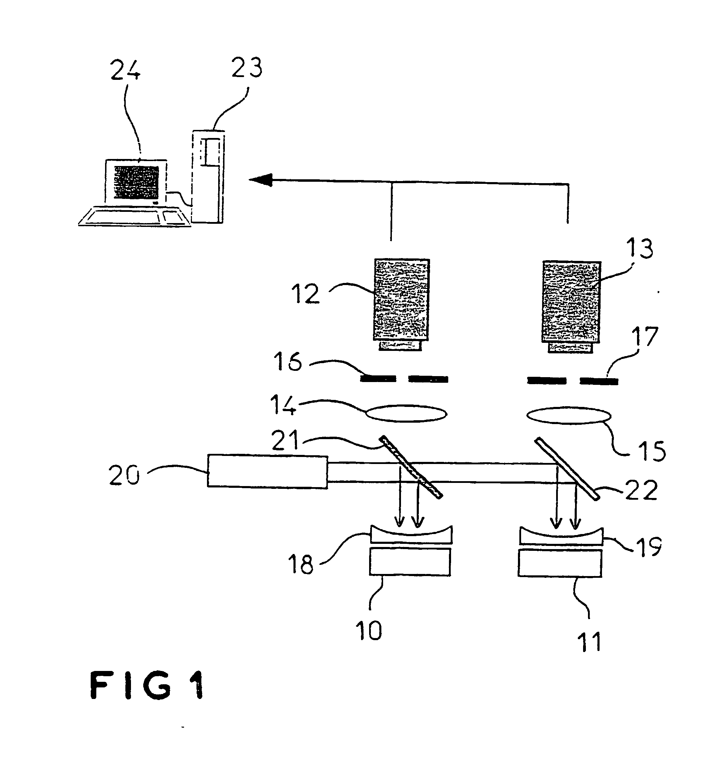

On a très schématiquement représenté sur la figure 1 une partie de l'installation objet de l'invention. En fait, la figure 1 représente la partie analyse et traitement, qui se situe donc en aval de la partie mécanique de séparation et de lavage décrite ci-après plus en détail.

Fondamentalement, cette partie analyse comporte deux canalisations du type veines (10) et (11), alimentées respectivement en filtrat concentré, également appelé jus, et en pâte lavée, essentiellement constituée de fibres en sortie de la partie mécanique ci-après décrite.

Le jus et les fibres circulent donc en continu au niveau de ces canalisations (10) et (11). Ces canalisations sont chacune pourvue d'une fenêtre transparente, au dessus de chacune desquelles est orientée de manière normale par rapport à celles-ci une caméra du type à transfert de charges, respectivement (12) et (13), éventuellement précédée d'éléments optiques appropriés, tels que lentilles (14) et (15) et diaphragmes (16) et (17), lesdits diaphragmes étant bien entendu réglables.

Par ailleurs, afin d'optimiser la surface analysée, lesdites fenêtres sont surmontées de lentilles plano-concaves (18, 19).

Ces fenêtres sont éclairées au moyen d'une source lumineuse impulsionnelle (20), typiquement un stroboscope, avantageusement un seul pour les deux veines, l'installation étant pourvue à cet effet d'une première lame séparatrice (21) et d'un miroir semi-transparent (22). La lame (21) et le miroir (22) sont en outre percés d'un orifice central (27), destiné à permettre l'acquisition par les caméras (12, 13) des données au niveau des fenêtres des veines (10, 11). Le fonctionnement des caméras (12) et (13) et du stroboscope (20) est géré par un ensemble électronique, qui synchronise le flash du stroboscope avec le fonctionnement des caméras, et notamment avec leur balayage trame.

Bien entendu, ce stroboscope peut être remplacé par tout système impulsionnel, voire par une couronne de diodes électroluminescentes (LED) suffisamment puissantes pour délivrer un niveau d'éclairement compatible avec des durées d'illumination très courtes. En effet, compte-tenu de la taille des particules à détecter, on souhaite aboutir à une résolution d'au moins trois microns, avec une vitesse de défilement de la pâte au sein des veines (10) et (11) de l'ordre de 1m/s, de sorte que la durée d'éclairage est de l'ordre de la microseconde.

Ainsi qu'on l'a représenté sur la figure 1, les deux caméras (12) et (13) sont reliées à une unité de traitement (23), typiquement un micro-ordinateur, au sein de laquelle sont stockées dans une mémoire appropriée les images numérisées captées par lesdites caméras. Chacune des images ainsi numérisées est traitée par l'unité (23), afin de détecter la présence des particules recherchées ainsi que leur taille. Ces particules sont en fait détectées par rétrodiffusion.

Ce traitement est géré par un logiciel, d'un type en soi connu pour cette application. Dans le cas d'espèce, cet ensemble de traitement (23) comporte deux entrées vidéo, qui sont analysées en quasi temps réel par ledit logiciel. On aboutit à un nombre de particules par unité de volume ainsi qu'à la surface de ces particules. La restitution de ces informations peut s'effectuer sur l'écran (24), par exemple sous la forme d'histogrammes. Par ailleurs, elles peuvent être imprimées au moyen d'une imprimante traditionnelle, voire transiter par une sortie de l'unité de traitement (23) sous forme de signaux analogiques ou numériques pour être exploités par tout système de traitement ainsi qu'il sera décrit ultérieurement.

L'ensemble caméras + flash est monté en roue libre par rapport à l'unité (23) à la cadence de 25 images/seconde, soit 50 trames/seconde.

Ainsi que déjà précisé, les caméras et le stroboscope ou système équivalent sont synchronisés entre eux. Ainsi, lorsque le traitement d'une image est terminé, ladite unité (23) ordonne à l'ensemble caméras - stroboscope, de lui restituer une nouvelle image numérisée des veines (10) et (11). De la sorte, les intervalles de temps séparant deux analyses successives sont réduits en vue d'une optimisation de la précision de l'installation par diminution de l'erreur statistique, compte-tenu de l'augmentation du nombre de mesures effectuées.

Par ailleurs et afin d'optimiser la détection par les caméras, la face des veines (10, 11) opposée aux fenêtres est avantageusement munie d'un fond approprié (25, 26), de couleur particulière, et choisi en fonction de l'opacité de la pâte et de la profondeur de champ des caméras.

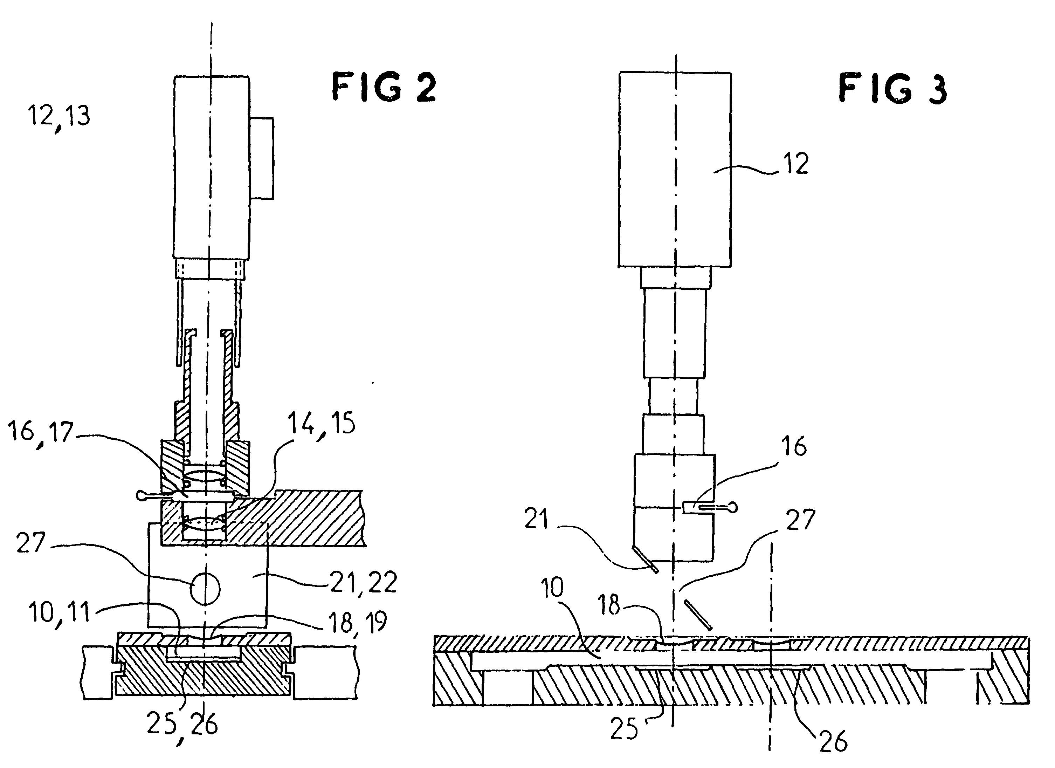

On a représenté sur les figures 2 et 3 une représentation plus détaillée en section respectivement transversale et longitudinale des caméras et des veines.

Il va être décrit maintenant plus en détail la partie mécanique de séparation et de lavage de la pâte, donc située en amont par rapport à la partie de traitement décrite précédemment. Cette partie mécanique comprend fondamentalement un tamis cylindrique micro-perforé (30), monté au niveau d'un carter (31), lui-même également de forme cylindrique. Ce tamis micro-perforé est destiné à permettre la séparation entre les fibres et les éléments fins contenus dans la pâte recyclée. Il est typiquement constitué d'une tôle réalisée en acier inoxydable de 1 ou 2 millimètres d'épaisseur, à orifices traversants de diamètre compris entre 0,1 et 0,3 millimètre, à entre-axe compris entre 1 et 3 millimètres. Dans un exemple de réalisation de l'invention, le diamètre interne du tamis cylindrique est de 120 millimètres pour une longueur de 200 millimètres.

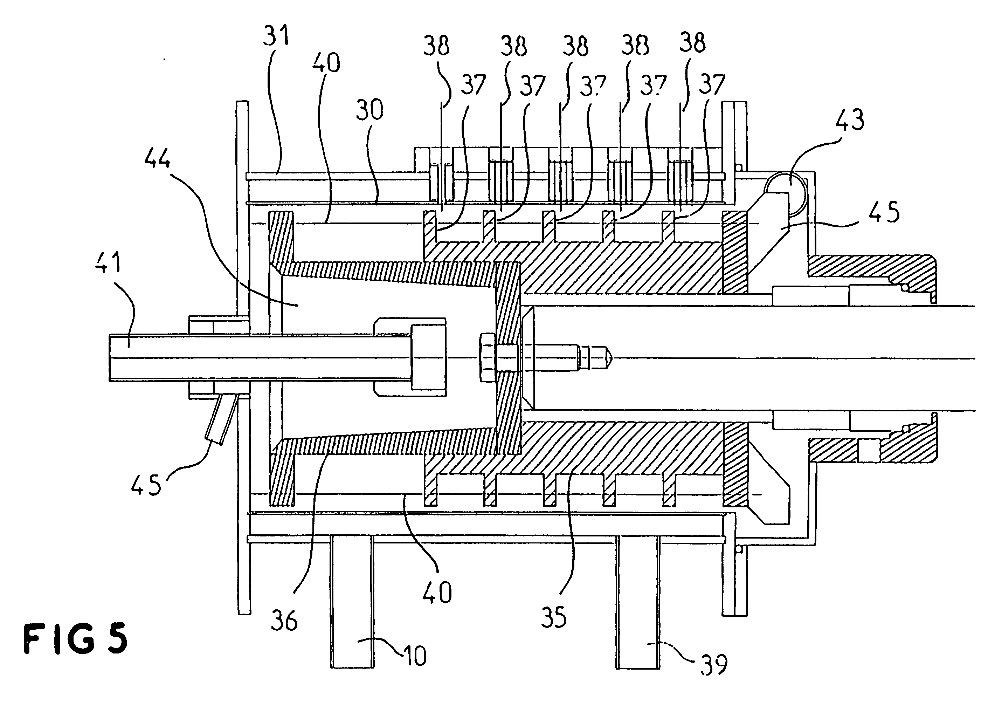

Un rotor (32) de décolmatage est animé d'un mouvement de rotation à l'intérieur du tamis (30) et ce, au moyen d'un moteur (33) extérieur au carter (31). Ce rotor comporte trois parties, une première partie centrale montée sur l'axe de sortie du moteur (33) à laquelle sont solidarisées une seconde partie (35), de révolution, et, à l'extrémité de la partie une troisième partie également de révolution (36), située sensiblement dans le prolongement de la partie (35), ainsi qu'on peut bien l'observer sur la figure 5.

Par ailleurs, la partie (35) est pourvue de disques de séparation radiaux (37) parallèles, destinés à délimiter des zones de lavage, de dilution et d'épaississement successives. En effet, le dispositif décrit comporte des entrées d'eau de dilution (38) au moyen de tubes fixés sur le carter (31) ménagés à la périphérie de celui-ci. Chacune de ces entrées (38) comporte en fait une buse d'injection d'eau de dilution à travers le tamis (30). Les disques de séparation (37) comportent chacun des encoches taillées en biais, ménagées en périphérie des disques (37), et inclinées par rapport au sens de rotation, de telle sorte à favoriser le passage de la pâte épaissie d'une zone de lavage à la suivante. Les filtrats dilués extraits au niveau des zones de lavage sont recyclés dans le processus de désencrage ainsi que les fractions après analyse. De fait, ces filtrats dilués sont collectés dans une canalisation (39), en vue de ce recyclage ultérieur.

Le rotor (32) comporte deux cylindres de décolmatage diamétralement opposés (40) traversant les disques de séparation (37) et représentés par leur axe (40) sur la figure 5.

La partie (36) du rotor (32) définit une chambre de désaération (44) au niveau de laquelle aboutit une canalisation (41) d'amenée de la pâte à papier à analyser. De fait, la canalisation (41) est alimentée directement en pâte à papier au cours de l'étape de désencrage. Cette pâte prélevée, présente une concentration d'environ 1 % et est diluée si nécessaire en amont du dispositif.

De fait, compte-tenu du positionnement de la canalisation (41), située selon l'axe de rotation du rotor (32), on assure ainsi un effet de pompage et de désaération de la pâte, compte-tenu en outre de la rotation du rotor et du volume de la chambre de désaération dimensionnée pour un temps de séjours suffisant. L'air est expulsé par le biais de la canalisation de sortie (45), ménagée au voisinage de la zone d'introduction de la canalisation (41) dans le carter (31).

La pâte est ensuite épaissie jusqu'à environ 3 % de concentration dans une première zone de classage à travers le tamis (30), du fait de la rétention des fibres. Les éléments fins contenant les fibres, les charges et les particules d'encre décrochées, traversent le tamis et alimentent l'une (10) des deux veines de mesure mentionnées dans le cadre de la description de la partie de traitement de installation. En l'absence de dilution, le filtrat issu de cette zone reste relativement concentré, ce qui évite de réduire le nombre de particules d'encre soumises à la mesure.

Le rotor (32) comporte des ailettes (45) de mise en pression de la pâte issue des zones de lavage. Une sortie tangentielle (43) est ménagée à l'opposé de la chambre de désaération (44), au niveau de laquelle transite la fraction de fibres lavées. Cette sortie tangentielle (43) alimente la seconde veine de mesure (11) des encres accrochées aux fibres. Cette fraction de pâte contient également des particules d'encre non liées aux fibres, mais retenues par le tamis, compte-tenu de leur taille. Cette fraction comporte donc des "points noirs", liés ou non à des fibres et ceci en quantité généralement très inférieure aux quantités d'encre accrochées aux fibres (encre couvrant la surface des fibres ou particules de différentes tailles fixées à des fibres selon le type d'encre).

Comme déjà dit, les éléments de décolmatage (40) du rotor sont constitués par des portions de cylindre dont l'axe (40), parallèle au tamis (30), traverse les différents disques de séparation (37) des sections de lavage. Le choix des cylindres de décolmatage en lieu et place de foils, c'est à dire d'éléments de décolmatage classiques de section en forme d'ailes d'avion, résulte de facilités de construction et de réglage.

Typiquement, le diamètre des cylindres de décolmatage est compris entre 6 et 12 mm, et l'entrefer entre les cylindres et le tamis est lui-même compris entre 1 et 3 mm.

Le diamètre du tamis est préférentiellement compris entre 100 et 150 millimètres pour une longueur comprise entre 150 et 300 millimètres. Ces dimensions permettent d'assurer les effets de pompage et de désaération de la pâte pour des débits de l'ordre de 0,1 m³/h à chacune des sorties (10, 39 et 43) et pour des vitesses de rotation de 1 000 à 2 000 tours/minute, selon le diamètre du rotor, et préférentiellement de 1 500 à 1 800 tours/minute pour 120 millimètre de diamètre.

Dans ces conditions, les pressions de pompage et de pulsation de pression de décolmatage varient de 0, 1 à 1 bars (10⁴ à 10⁵ Pascals), préférentiellement de 0,2 à 0,7 bars (2.10⁴ à 7.10⁴ Pascals). La durée de séjour de la pâte dans la chambre de désaération (44) est de l'ordre de 10 secondes, et les accélérations centripètes de quelques centaines de m/s², ce qui permet d'assurer la séparation de bulles d'air jusqu'à environ 30 mm de diamètre, c'est à dire d'éliminer l'essentiel des bulles d'air restant dans la pâte notamment après flottation, lesdites bulles s'évacuant par la sortie (45).

Compte-tenu de la dimension des perforations du tamis (30), celui-ci ne laisse passer que les éléments fins (fines, charges et encres) et retient les fibres et ce, contrairement aux dispositifs traditionnels de classage, qui visent à laisser passer les fibres et à retenir les contaminants.

Dans une autre forme de réalisation de l'invention, les disques de séparation (37) des zones de lavage sucessives sont remplacés par une hélice, dont le pas et l'extension radiale correspondent à ceux des disques, ce qui conduit à un lavage continu par dilutions et épaississements successifs le long du canal hélicoïdal ainsi formé.

Le sens d'inclinaison de hélice est défini par le sens de rotation du rotor de manière à assurer l'évacuation de la pâte vers la sortie de fibres lavées (43) sous l'effet du frottement de la suspension à la surface du tamis.

Avantageusement, de manière à réduire la consommation d'eau de lavage, la chambre annulaire externe au tamis (30) comporte une cloison spiralée définissant un canal hélicoïdal de type analogue à celui du rotor (32), mais fixe et situé en aval du tamis (30). La sortie de filtrats dilués est supprimée et les alimentations d'eau (38) sont remplacées par une seule alimentation d'eau de lavage au niveau de l'extrémité du canal spiralé fixe situé du coté de la sortie (43) de fibres lavées.

Dans ces conditions, les filtrats traversant le tamis en phase de surpression sont repompés à travers le tamis en phase de dépression créée par les cylindres de décolmatage (40) du rotor, ce qui conduit à un lavage à contre-courant des fibres en amont du tamis (30) par l'eau de dilution puis les filtrats de moins en moins dilués, circulant dans le canal spiralé fixe vers la sortie des filtrats concentrés (10). Avantageusement, le rotor est équipé d'un plus grand nombre de cylindres de décolmatage (40) de manière à augmenter la durée des phases de dépression.

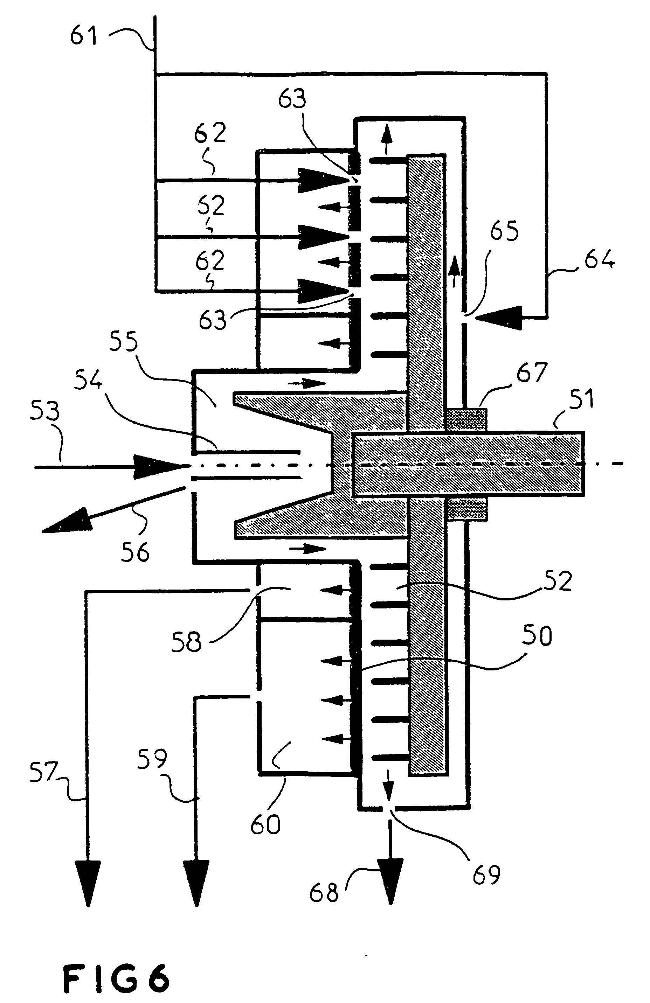

Selon une autre forme de réalisation de invention, représentée sur la figure 6, le tamis de séparation peut être plan et se présenter sous la forme d'un disque plan micro-perforé (50). Le rotor (51) peut également être plan et comporter un canal spiralé (52) assurant l'écoulement centrifuge de la pâte puis des fibres lavées.

Le dispositif est alimenté en (53) dans l'axe du rotor (51) par un tube (54) débouchant dans une chambre (55) de manière à permettre l'élimination de l'air en (56), dans des conditions analogues à celles décrites précédemment.

La pâte est ensuite entraînée vers le canal spiralé (52) qui assure également l'effet de pompage et de décolmatage du tamis (50).

Les filtrats concentrés sont extraits en (57) de la chambre annulaire (58) et les filtrats dilués en (59) de la chambre annulaire (60).

L'eau de dilution est introduite en (61), d'une part en différents points (62) par l'intermédiaire de tubes (non représentés) traversant la chambre (60) et le tamis (50) par des orifices (63), et d'autre part en (64) par un orifice (65) ménagé dans la paroi près du dispositif d'étanchéité (67) de manière à éviter l'accumulation de pâte à l'arrière du rotor et à diluer les fibres lavées extraites en (68) par une sortie tangentielle (69).

Avantageusement de manière à réduire la consommation d'eau de lavage, la cloison cylindrique de séparation entre les chambres annulaires (58) et (60) est remplacée par une cloison spiralée, de type analogue à celle définissant le canal spiralé du rotor (51), mais fixe et située en aval du tamis (50). La sortie de filtrats dilués (59) est supprimée et les alimentations d'eau (62) sont remplacées par une seule alimentation d'eau de lavage au niveau de l'extrémité périphérique du canal spiralé fixe en aval du tamis (50).

Dans ces conditions, les filtrats traversant le tamis en phase de surpression sont repompés à travers le tamis en phase de dépression crée par le rotor, ce qui conduit à un lavage à contre courant des fibres en amont du tamis (50) par l'eau de dilution puis les filtrats de moins en moins dilués, circulant dans le canal spiralé fixe vers la sortie de filtrats concentrés (57).

Il va être décrit ci-après différentes applications de l'invention, notamment dans le cadre de la régulation d'une chaîne de désencrage de pâtes à papier issues de papiers recyclés ou de récupération.

De nombreuses études ont pu montrer que les paramètres influençant le décrochage de l'encre lors de la mise en pâte, c'est à dire au niveau du pulpeur et du traitement à chaud (triturateur à chaud ou disperseur), sont essentiellement constitués par le mode d'action (type de matériel et concentration de la pâte), l'énergie spécifique, la température, les adjuvants chimiques et bien sûr le type d'encre.

On a également démontré que les paramètres influençant l'élimination de l'encre par flottation ou par lavage dépendent essentiellement de la technologie utilisée, de la physico-chimie du milieu et des caractéristiques des encres (propriétés de surface et granulométrie).

Compte-tenu de la multiplicité de ces paramètres, de nombreuses applications du dispositif conforme à l'invention sont envisageables. En effet, le dispositif de séparation et de contrôle continu des fractions d'encre peut avantageusement être combiné à une mesure de blancheur (par des moyens connus) sur chacune des fractions.

De fait, les exemples décrits ci-après illustrent, pour différentes implantations du dispositif de contrôle continu des encres accrochées et décroches, quelques possibilités de régulation de certaines étapes essentielles du désencrage.

### Exemple I : Implantation du dispositif de contrôle en début de chaîne de désencrage

Le dispositif ne peut être implanté en début de chaîne qu'après mise en pâte des vieux papiers et classage grossier, c'est à dire au niveau de la pré- épuration à partir du cuvier de vidange du pulpeur (en sortie de pré-classage à trous, continu ou discontinu selon le type de pulpeur). Une implantation en fin de pré-épuration présente l'avantage de faciliter le prélèvement continu de pâte (concentration plus faible) et d'éviter les risques de bouchage du dispositif de séparation des fractions d'encres, par des contaminants grossiers.

Dans ces conditions d'implantation, l'action en amont sur les conditions de pulpage ne peut être envisagée qu'avec une durée de retard correspondant sensiblement à la durée de pulpage. Malgré ce retard, la puissance spécifique de pulpage, notamment la durée de pulpage, et les adjuvants peuvent être régulés en fonction des quantités d'encre accrochée et de la fragmentation des encres décrochées.

Ainsi, lorsqu'un lot de balles de vieux papiers comporte des encres difficiles à décrocher, le dispositif de contrôle conforme à l'invention permet d'augmenter la puissance spécifique de pulpage et/ou l'alcalinité de manière à améliorer le décrochage, en particulier lorsque l'installation de désencrage ne comporte pas de traitement à chaud.

Au contraire, lorsqu'un lot de vieux papiers comporte des encres facilement détachables et/ou dispersables (encres de type hydrosoluble issues notamment de journaux imprimés en flexographie) la détection d'encres détachées et/ou fortement fragmentées permet de prendre les mesures inverses au niveau du pulpage.

### Exemple II : Implantation du dispositif de contrôle en premier stade de désencrage

L'analyse de la pâte après désencrage par flottation ou lavage, c'est à dire au niveau de l'épuration fine, permet un contrôle plus précis des fractions d'encre du fait que l'essentiel de l'encre, décrochée au pulpage, a été éliminée au désencrage.

A ce niveau, la présence résiduelle de particules d'encre dans les filtrats du dispositif de séparation détectée par le dispositif de contrôle conforme à l'invention indique une efficacité de flottation ou de lavage insuffisante. Les possibilités de régulation du désencrage dépendent des technologies utilisées ainsi que du dimensionnement des équipements.

La régulation de la flottation à partir du contrôle de l'encre décrochée peut être basée, soit sur la commande de l'adjonction de produits de désencrage, soit sur la commande des paramètres essentiels de la flottation en particulier le taux d'air, la taille des bulles, la concentration, le taux de recyclage et le taux de rejets. Toutefois, ces paramètres ne sont pas indépendants et ne peuvent varier que dans certaines limites définies par le type et le dimensionnement des matériels.

A titre d'exemple, les paramètres technologiques régulés peuvent être le taux de refus (mousses) et/ou le taux de recirculation autour des cellules de flottation si le dimensionnement de l'installation est suffisant.

Une augmentation du débit d'alimentation, et donc du taux de recirculation à concentration et production constantes, conduit, dans le cas le plus courant de cellules à injecteurs, à une augmentation de la pression d'alimentation et du débit d'air, ce qui améliore l'efficacité de désencrage au détriment du rendement matière et de la consommation énergétique.

Une augmentation du taux de refus volumique, conduit à une amélioration complémentaire de l'efficacité de désencrage. A noter que cet accroissement du taux de refus des cellules primaires, implantées en ligne, implique que la capacité des cellules secondaires, traitant les refus des cellules primaires, soit suffisante et/ou que soit augmentée la fraction de refus primaires non traités, c'est à dire les mousses des premières cellules primaires qui sont en général rejetées car très chargées en encre. Ces conditions réduisent le rendement de manière significative.

La régulation de la concentration de flottation constitue une autre possibilité intéressante d'amélioration de l'efficacité de désencrage, dans la mesure où une réduction de la concentration diminue les pertes, ce qui permet, si nécessaire, d'augmenter les débits et le taux de recirculation sans affecter le rendement.

Ce mode de régulation exige cependant des matériels de capacité suffisante, notamment au niveau de l'épaississement qui limite fréquemment la production. En d'autres termes, il s'agirait alors de réduire la production pour améliorer la qualité.

Avantageusement, pour certains matériels d'épaississement de la pâte, il est possible de réduire la rétention des éléments fins et des petites particules d'encre décrochées qui sont ainsi recyclées en amont des cellules de flottation, ce qui améliore l'efficacité de désencrage au détriment du rendement. En d'autres termes, ce mode de régulation du fonctionnement des matériels d'épaississement revient à compléter la flottation par un lavage partiel de la pâte.

La régulation du lavage à partir du contrôle de l'encre décrochée peut être basée, soit sur la commande des paramètres agissant sur la sélectivité du lavage (rétention des éléments fins et des encres déterminée par les conditions physico-chimiques et hydrodynamiques en amont du tamis ou de la toile), soit sur la commande du taux de lavage, c'est à dire du facteur d'épaississement de la pâte. Les possibilités de régulation du lavage sont ainsi nombreuses et particulières à chacune des diverses technologies de lavage actuellement utilisées.

A titre d'exemple, le lavage sur matériels d'épaississement sélectif sur toiles dérivés de machines à papier, peut être régulé par la commande de la vitesse de défilement des toiles, ce qui conduit pour une production de pâte lavée constante à des variations du grammage de fibres déposées sur la toile, et donc des variations de rétention des encres et éléments fins. Dans ce cas, et d'une manière générale, l'amélioration du désencrage sera obtenue au détriment du rendement.

Les possibilités de régulation du désencrage à partir du contrôle des encres dans la fraction des fibres lavées du dispositif de séparation concernent surtout d'autres étapes du processus. Les encres mesurées dans cette fraction correspondent pour l'essentiel à des encres accrochées aux fibres, ainsi qu'à des points noirs (particules d'encre de dimension "visible") pouvant être liés ou non à des fibres.

Le contrôle des points noirs peut intervenir dans la régulation des étapes de désencrage permettant de les éliminer, à savoir principalement la flottation, le cyclonage et les traitements à chaud en disperseur ou en triturateur.

En ce qui concerne la flottation et le cyclonage, une régulation basée sur la commande de la concentration est particulièrement efficace dans la mesure où la dilution en flottation et au cyclonage, ainsi que l'augmentation des pertes de charge qui en résulte notamment au cyclonage, sont les principaux paramètres de fonctionnement permettant d'améliorer l'efficacité d'élimination des points noirs.

En ce qui concerne le traitement à chaud, les paramètres technologiques permettant d'améliorer la dispersion des points noirs sont essentiellement les mêmes que ceux qui permettent d'améliorer le décrochage des encres.

La régulation de la dispersion ou de la trituration à chaud à partir du contrôle des encres accrochées peut avantageusement être basée sur la commande de la puissance spécifique, dans la mesure où la consommation énergétique du traitement est élevée, soit 30 à 100 kWh/t pour le disperseur ou le triturateur, hors énergie spécifique nécessaire au chauffage et à l'épaississement de la pâte.

L'économie d'énergie obtenue par régulation d'un traitement à chaud dépend des variations des compositions des matières premières. L'économie peut atteindre 20 à 50 kWh/t lorsque les lots de vieux papiers traités ne comportent des encres difficiles à décrocher que de manière épisodique, ce qui permet de travailler la plupart du temps à faible puissance spécifique au niveau d'un traitement à chaud régulé en fonction des quantités d'encre mesurées dans la fraction de fibres lavées du dispositif de séparation. Dans ces conditions, la régulation du traitement à chaud permet également d'éviter, la plupart du temps, certaines dégradations des fibres observées aux fortes puissances spécifiques nécessaires pour assurer le décrochage des encres les plus difficiles.

La régulation de la température du traitement à chaud, en fonction des quantités d'encre accrochée, offre également des possibilités d'économie d'énergie, mais l'optimisation de ce paramètre est plus délicate, dans la mesure où si le décrochage d'encres de type thermofusible est souvent amélioré à température élevée, leur fragmentation est généralement moins importante.

La régulation de la quantité d'adjuvants chimiques introduits au traitement à chaud ne concerne en principe que les installations comportant un stade de blanchiment en aval du triturateur ou du disperseur, faisant alors fonction de mélangeur et partiellement de réacteur pour les produits de blanchiment. Ce mode de régulation du traitement à chaud en combinaison avec le blanchiment peut être effectué à partir du contrôle d'encre accrochée, soit en amont du traitement à chaud au niveau du premier stade de désencrage, soit en aval de ce traitement, en particulier au niveau d'un second stade de désencrage.

Avantageusement, la régulation combinée du traitement à chaud et du blanchiment peut être basée sur le contrôle, d'une part de l'encre accrochée et, d'autre part, de la blancheur et si nécessaire de la couleur, mesurés sur la fraction de fibres lavées du dispositif de séparation.

La mesure des composantes de la couleur (coordonnées Lab : représentation de l'espace des couleurs, calculée à partir du spectre de réflectance obtenu avec une résolution de 10 à 20 nm) permet également d'adapter le type de blanchiment ultérieur à la coloration de la pâte. Ainsi une blancheur insuffisante des fibres peut être corrigée, soit par une augmentation de la dose d'agents de blanchiment ou par la mise en oeuvre d'un autre type de blanchiment (notamment hydrosulfite ou FAS (Acide Formamidine Sulfinique)) adapté à la décoloration de la pâte, lorsque l'encre est décrochée des fibres (ce qui favorise également le décrochage dans les cas d'un blanchiment en milieu alcalin, notamment au peroxyde d'hydrogène), soit par une augmentation de la puissance spécifique du traitement à chaud, lorsque les encres sont insuffisamment décrochées des fibres et si l'amélioration du décrochage de l'encre est suivi de son élimination en second stade de désencrage.

### Exemple III : Implantation du dispositif de contrôle en second stade de désencrage

L'analyse de la pâte après un premier stade de désencrage, c'est à dire après élimination de la plupart de l'encre par flottation et/ou lavage et décrochage de l'encre par traitement à chaud, suivi en général d'un premier stade de blanchiment, permet de contrôler plus particulièrement les encres résiduelles difficiles à décrocher et/ou insuffisamment éliminées.

Avantageusement, le dispositif de contrôle continu des encres accrochées et décrochées peut être implanté au niveau du post-désencrage, c'est à dire après dilution en sortie de blanchiment, de manière à faciliter les conditions de prélèvement de la pâte et à bénéficier des possibilités de contrôle combiné du blanchiment et du traitement à chaud.

A ce niveau, les possibilités de régulation du post-désencrage à partir du contrôle des encres accrochées et décrochées sont analogues à celles décrites précédemment, avec des possibilités supplémentaires de régulation en amont et en aval de la mesure pour des installations de désencrage sophistiquées comportant deux stades de blanchiment et de traitement à chaud.

Pour l'ensemble de ces possibilités de régulation de différentes étapes de désencrage, basées sur le contrôle des encres accrochées et décrochées, les grandeurs prises en compte pour la régulation sont le nombre ou la surface des particules d'encre mesurées par unité de volume de suspension analysée (ou par unité de masse de matières en suspension) sur l'ensemble ou sur certaines classes de tailles des particules d'encre.

Avantageusement, le contrôle peut être basé sur la mesure des surfaces de particules d'encre mesurées dans deux classes de tailles, de manière à commander différentes étapes d'élimination ou de décrochage des encres à partir d'un ensemble de mesures des divers types d'encre, selon les possibilités de régulation décrites :
- dans les filtrats du dispositif de séparation ; mesure des petites et des grosses particules d'encre décrochée, avec une limite entre les deux classes de tailles se situant de préférence entre 5 et 20 mm ;
- dans les fibres lavées du dispositif de séparation ; mesure des petites particules d'encre accrochées aux fibres et des grosses particules d'encre (points noirs pour les plus grosses) liées ou non à des fibres, avec une limite entre les deux classes de taille se situant de préférence entre 50 et 200 mm.

Le dispositif de séparation peut être utilisé, indépendamment ou non du dispositif de contrôle des encres, pour la régulation d'opérations de blanchiment ou de décoloration, à partir de mesures de réflectance sur la fraction de fibres lavées (mesures de blancheur et de couleur des fibres non affectées par la coloration et la blancheur des eaux et des éléments fins) au niveau des circuits de préparation de pâtes neuves ou recyclées, ou au niveau de circuits de machine à papier incluant le traitement des cassés de fabrication.

Il ressort de ces différents exemples tout l'intérêt du dispositif de contrôle de l'invention dans la régulation d'une chaîne de désencrage, notamment en termes d'efficacité, mais également en termes de coûts.

## Revendications

1. Dispositif pour mesurer en continu la quantité de particules d'encre en suspension dans une pâte à papier issue de papiers recyclés se présentant sous forme décrochées, c'est à dire individualisées au sein de la pâte, et sous forme accrochées, c'est à dire liées à des fibres papetières comprenant :
- un ensemble de séparation et de lavage (30 - 33) des particules d'encre en fractions respectivement accrochées et décrochées, ledit ensemble étant alimenté en continu en pâte à papier par une canalisation appropriée (41), et comprenant :
• un tamis micro-perforé cylindrique (30) à l'intérieur duquel est acheminée en continu par une canalisation (41) la pâte à papier à analyser ;
• un organe de décolmatage et de classage (32), destiné à assurer la création de pulsations de pression, et à entraîner la pâte à papier à la surface du tamis (30) ;
• des canalisations de collecte et de sortie, respectivement (10) des particules d'encres décrochées non diluées, des éléments fins dilués (39), et des fibres papetières lavées avec particules d'encre accrochées (43) ;
- un ensemble de mesure et de traitement, comprenant :
• deux veines (10, 11), issues dudit ensemble de séparation, acheminant lesdites fractions d'encre au niveau d'une zone de mesure et comportant chacune au niveau de cette zone une fenêtre transparente ;
• un organe de mesure et d'acquisition comprenant :
◆ un organe d'éclairage (20) desdites fenêtres ;
◆ deux caméras (12, 13) situées en regard de chacune desdites fenêtres, synchronisées avec l'organe d'éclairage (20) et destinées à acquérir à intervalles réguliers une image des fenêtres ;
• des moyens de numérisation et de traitement (23) des images ainsi acquises, destinés à déterminer la dimension et la quantité desdites particules d'encre;
• un organe de sortie des données ainsi déterminées, notamment vers un organe de restitution sensorielle (24), ou sous la forme de signaux analogiques ou numériques, destinés à actionner un ou plusieurs organes déterminés.

2. Dispositif selon la revendication 1, ***caractérisé* en ce que** :
- l'ogane de décolmatage et de classage est constitué par un rotor (32), dont l'axe de rotation est colinéaire avec l'axe du tamis ;
- la canalisation de collecte et de sortie des fibres papetières lavées avec particules d'encre accrochées (43) est située au sein du volume interne défini par le tamis (30).

3. Dispositif selon la revendication 2, ***caractérisé* en ce que** le rotor (32) définit en amont de l'ensemble de séparation et de lavage une chambre de désaération (44), au niveau de laquelle aboutit la canalisation (41) d'acheminement de la pâte à papier à analyser, ladite chambre de désaération communiquant avec l'extérieur par un évent (45) d'évacuation des bulles d'air contenues dans ladite pâte.

4. Dispositif selon l'une des revendications 2 et 3, ***caractérisé* en ce que** le rotor est muni sur sa surface périphérique externe de disques radiaux (37) au niveau desquels aboutissent des canalisations acheminant l'eau de lavage, lesdits disques étant équipés d'ouvertures de contrôle de flux de la pâte à papier en amont du tamis (30).

5. Dispositif selon l'une des revendications 2 et 3, ***caractérisé* en ce que** le rotor est muni à sa périphérie d'une hélice spiralée au niveau de laquelle aboutissent des canalisations acheminant l'eau de lavage.

6. Dispositif selon l'une des revendications 2 à 5, ***caractérisé* en ce que** le rotor (30) est muni de cylindres de décolmatage (40), ménagés au voisinage de sa périphérie, et s'étendant parallèlement à l'axe de rotation du rotor.

7. Dispositif selon l'une des revendications 2 à 6, ***caractérisé* en ce que** le tamis (30) comporte en aval un canal spiralé fixe, destiné à effectuer un lavage des fibres à contre-courant.

8. Dispositif pour mesurer en continu la quantité de particules d'encre en suspension dans une pâte à papier issue de papiers recyclés se présentant sous forme décrochées, c'est à dire individualisées au sein de la pâte, et sous forme accrochées, c'est à dire liées à des fibres papetières comprenant :
- un ensemble de séparation et de lavage (30 - 33) des particules d'encre en fractions respectivement accrochées et décrochées, ledit ensemble étant alimenté en continu en pâte à papier par une canalisation appropriée (41), et comprenant :
• un tamis micro-perforé plan (50) au niveau duquel est acheminée en continu par une canalisation (53) la pâte à papier à analyser ;
• un organe de décolmatage et de classage (51), destiné à assurer la création de pulsations de pression, et à entraîner la pâte à papier à la surface du tamis (50) ;
• des canalisations de collecte et de sortie, respectivement (57) des particules d'encres décrochées non diluées, des éléments fins dilués (59), et des fibres papetières lavées avec particules d'encre accrochées (68) ;
- un ensemble de mesure et de traitement, comprenant :
• deux veines (10, 11), issues dudit ensemble de séparation, acheminant lesdites fractions d'encre au niveau d'une zone de mesure et comportant chacune au niveau de cette zone une fenêtre transparente ;
• un organe de mesure et d'acquisition comprenant : organe de mesure et d'acquisition comprenant :
◆ un organe d'éclairage (20) desdites fenêtres ;
◆ deux caméras (12, 13) situées en regard de chacune desdites fenêtres, synchronisées avec l'organe d'éclairage (20) et destinées à acquérir à intervalles réguliers une image des fenêtres ;
• des moyens de numérisation et de traitement (23) des images ainsi acquises, destinés à déterminer la dimension et la quantité desdites particules d'encre ;
• un organe de sortie des données ainsi déterminées, notamment vers un organe de restitution sensorielle (24), ou sous la forme de signaux analogiques ou numériques, destinés à actionner un ou plusieurs organes déterminés.

9. Dispositif selon la revendication 8, ***caractérisé* en ce que** l'organe de décolmatage et de classage (51) est constitué par un rotor comportant un canal spiralé, animé d'un mouvement de rotation, et dont l'axe de rotation est confondu avec le centre du tamis (50), la rotation dudit rotor assurant le pompage de la pâte à papier en amont du tamis et définissant une chambre de désaération de ladite pâte au niveau de sa zone centrale.

10. Dispositif selon la revendication 8, ***caractérisé* en ce que** le tamis (50) comporte en aval un canal spiralé fixe, destiné à effectuer un lavage des fibres à contre-courant.

11. Procédé de régulation d'une chaîne de désencrage de pâte à papier issue de papiers recyclés consistant à mesurer en continu la quantité des particules d'encre en suspension individualisées au sein de la pâte et la quantité des particules d'encre en suspension liées à d'autres particules plus volumineuses, et notamment aux fibres papetières, au moyen d'un dispositif selon l'une des revendications 1 ou 8, puis en fonction des mesures ainsi effectuées, à agir au niveau des différents paramètres de fonctionnement de la chaîne de désencrage, notamment lors des étapes de pulpage et de trituration à chaud, et lors des étapes de lavage et de flottation.

12. Procédé de régulation d'une chaîne de désencrage de pâte à papier issue de papiers recyclés selon la revendication 11, ***caractérisé* en ce que** la mesure des particules d'encre accrochées et décrochées intervient en début de chaîne de désencrage, et/ou après une première étape de désencrage, c'est à dire après flottation et/ou lavage, et/ou après une seconde étape de désencrage.

13. Procédé de régulation d'une chaîne de désencrage de pâte à papier issue de papiers recyclés selon l'une des revendications 11 et 12, ***caractérisé* en ce que** le contrôle des quantités respectives des particules d'encre décrochées et d'encre accrochées est couplé à un contrôle de réflectance dans le cadre de l'affinement de la régulation des étapes de décrochage, de fragmentation de l'encre, de blanchiment et/ou de décoloration de la pâte.

## Patentansprüche

1. Vorrichtung zum kontinuierlichen Messen der Menge von Tintenpartikeln in Suspension in einem von rezyklierten Papieren stammenden Ganzstoff, die in abgelöster Form, d.h. innerhalb des Ganzstoffs getrennt, und in anhaftender Form, d.h. an Papierfasern gebunden, vorhanden sind, umfassend:
- eme Anlage zum Trennen und Waschen (30-33) der Tintenpartikel in anhaftenden bzw. abgelösten Fraktionen, wobei die Anlage kontinuierlich mit Ganzstoff durch einen geeigneten Kanal (41) gespeist wird und umfassend:
• ein zylindrisches Sieb (30) mit Mikroperforationen, von dem kontinuierlich durch einen Kanal (41) der zu analysierende Ganzstoff befördert wird;
• ein Element zum Reinigen und Klassieren (32), das dazu bestimmt ist, die Erzeugung von Druckimpulsen sicher zu stellen und den Ganzstoff an die Oberfläche des Siebs (30) zu treiben;
• Kanäle zum Sammeln und Ableiten der nicht verdünnten abgelösten Tintenpartikel, der feinen verdünnten Partikel (39) bzw. (10) der gewaschenen Papierfasern mit anhaftenden Tintenpartikeln (43);
- eine Mess- und Bearbeitungsanlage, umfassend:
• zwei Stränge (10, 11), die aus der Trennanlage kommen und die Tintenfraktionen in den Bereich einer Messzone leiten und jeweils im Bereich dieser Zone ein transparentes Fenster umfassen;
• ein Mess- und Erfassungselement, umfassend:
◆ ein Element (20) zum Beleuchten der Fenster;
◆ zwei Kameras (12, 13), die gegenüber jedem der Fenster angeordnet und mit dem Beleuchtungselement (20) synchronisiert und dazu bestimmt sind, in regelmäßigen Intervallen ein Bild der Fenster zu erfassen;
• Digitalisierungs- und Bearbeitungsmittel (23) für die so erfassten Bilder, die dazu bestimmt sind, die Abmessung und Menge der Tintenpartikel zu bestimmen;
• ein Element zur Ausgabe der so bestimmten Daten, insbesondere zu einem sensorischen Wiedergabeelement (24), oder in Form von Analog- oder Digitalsignalen, die dazu bestimmt sind, ein oder mehrere bestimmte Elemente zu betätigen.

2. Vorrichtung nach Anspruch 1, **dadurch gekennzeichnet, dass**:
- das Element zum Reinigen und Klassieren von einem Rotor (32) gebildet ist, dessen Rotationsachse mit der Achse des Siebs kolinear ist;
- der Kanal zum Sammeln und Ableiten der gewaschenen Papierfasern mit anhaftenden Tintenpartikeln (43) innerhalb des von dem Sieb (30) definierten Innenvolumens angeordnet ist.

3. Vorrichtung nach Anspruch 2, **dadurch gekennzeichnet, dass** der Rotor (32) stromaufwärts zur Trenn- und Waschanlage eine Entlüftungskammer (44) definiert, m der der Kanal (41) zur Beförderung des zu analysierenden Ganzstoffs mündet, wobei die Entlüftungskammer mit der Umgebung durch ein Luftloch (45) zur Beseitigung der in der Suspension enthaltenen Luftblasen verbunden ist.

4. Vorrichtung nach einem der Ansprüche 2 und 3, **dadurch gekennzeichnet, dass** der Rotor auf seiner äußeren Umfangsfläche mit Radialscheiben (37) versehen ist, in deren Bereich Kanäle münden, die das Waschwasser befördern, wobei diese Scheiben mit Öffnungen zur Kontrolle des Flusses des Ganzstoffs stromaufwärts zum Sieb (30) versehen sind.

5. Vorrichtung nach einem der Ansprüche 2 und 3, **dadurch gekennzeichnet, dass** der Rotor an seinem Umfang mit einer Spiralschraube versehen ist, in deren Bereich Kanäle münden, die das Waschwasser befördern.

6. Vorrichtung nach einem der Ansprüche 2 bis 5, **dadurch gekennzeichnet, dass** der Rotor (30) mit Reinigungszylindern (40) versehen ist, die in der Nähe seines Umfangs vorgesehen sind und sich parallel zur Rotationsachse des Rotors erstrecken.

7. Vorrichtung nach einem der Ansprüche 2 bis 6, **dadurch gekennzeichnet, dass** das Sieb (30) stromabwärts einen festen spiralförmigen Kanal umfasst, der dazu bestimmt ist, ein Waschen der Fasern mit Gegenstrom durchzuführen.

8. Vorrichtung zum kontinuierlichen Messen der Menge von Tintenpartikeln in Suspension in einem von rezyklierten Papieren stammenden Ganzstoff, die in abgelöster Form, d.h. innerhalb des Ganzstoffs getrennt, und in anhaftender Form, d.h an Papierfasern gebunden, vorhanden sind, umfassend:
- eine Anlage zum Trennen und Waschen (30-33) der Tintenpartikel in anhaftenden bzw. abgelösten Fraktionen, wobei die Anlage kontinuierlich mit Ganzstoff durch einen geeigneten Kanal (41) gespeist wird und umfassend:
• ein zylindrisches Sieb (50) mit Mikroperforationen, von dem kontinuierlich durch einen Kanal (53) der zu analysierende Ganzstoff befördert wird;
• ein Element zum Reinigen und Klassieren (51), das dazu bestimmt ist, die Erzeugung von Druckimpulsen sicher zu stellen und den Ganzstoff an die Oberfläche des Siebs (50) zu treiben;
• Kanäle zum Sammeln und Ableiten der nicht verdünnten abgelösten Tintenpartikel, der feinen verdünnten Partikel (59) bzw. (57) der gewaschenen Papierfasern mit anhaftenden Tintenpartikeln (68);
- eine Mess- und Bearbeitungsanlage, umfassend:
• zwei Stränge (10, 11), die aus der Trennanlage kommen und die Tintenfraktionen in den Bereich einer Messzone leiten und jeweils im Bereich dieser Zone ein transparentes Fenster umfassen;
• ein Mess- und Erfassungselement, umfassend:
◆ ein Element (20) zum Beleuchten der Fenster;
◆ zwei Kameras (12, 13), die gegenüber jedem der Fenster angeordnet und mit dem Beleuchtungselement (20) synchronisiert und dazu bestimmt sind, in regelmäßigen Intervallen ein Bild der Fenster zu erfassen;
• Digitalisierungs- und Bearbeitungsmittel (23) für die so erfassten Bilder, die dazu bestimmt sind, die Abmessung und Menge der Tintenpartikel zu bestimmen;
• ein Element zur Ausgabe der so bestimmten Daten, insbesondere zu einem sensorischen Wiedergabeelement (24), oder in Form von Analog- oder Digitalsignalen, die dazu bestimmt sind, ein oder mehrere bestimmte Elemente zu betätigen.

9. Vorrichtung nach Anspruch 8, **dadurch gekennzeichnet, dass** das Element zum Reinigen und Klassieren (51) von einem Rotor gebildet ist, der einen spiralförmigen Kanal umfasst, der eine Drehbewegung ausführt und dessen Rotationsachse mit dem Zentrum des Siebes (50) zusammenfällt, wobei die Drehung des Rotors das Pumpen des Ganzstoffs stromaufwärts zum Sieb sicher stellt, und eine Entlüftungskammer für den Ganzstoff im Bereich seiner zentralen Zone definiert.

10. Vorrichtung nach Anspruch 8, **dadurch gekennzeichnet, dass** das Sieb (50) stromabwärts einen festen spiralförmigen Kanal umfasst, der dazu bestimmt ist, ein Waschen der Fasern mit Gegenstrom durchzuführen.

11. Regelverfahren für eine Anlage zum Deinken eines Ganzstoffs aus rezyklierten Papieren, das darin besteht, kontinuierlich die Menge der Tintenpartikel in Suspension, die in dem Ganzstoff getrennt vorhanden sind, und die Menge der Tintenpartikel in Suspension, die in dem Ganzstoff an andere voluminösere Partikel, insbesondere an die Papierfasern, gebunden sind, mit Hilfe einer Vorrichtung nach einem der Ansprüche 1 bis 8 zu messen, dann in Abhängigkeit von den so durchgeführten Messungen im Bereich der verschiedenen Funktionsparameter der Anlage zum Deinken einzuwirken, insbesondere bei den Schritten der Breiherstellung und des Heißmahlens und bei den Schritten des Waschens und der Flotation.

12. Regelverfahren für eine Anlage zum Deinken eines Ganzstoffs aus rezyklierten Papieren nach Anspruch 11, **dadurch gekennzeichnet, dass** das Messen der anhaftenden und abgelösten Tintenpartikel am Beginn der Anlage zum Deinken und/oder nach einem ersten Schritt des Deinkens, d.h. nach der Flotation und/oder dem Waschen und/oder nach einem zweiten Schritt des Deinkens erfolgt.

13. Regelverfahren für eine Anlage zum Deinken eines Ganzstoffs aus rezyklierten Papieren nach Anspruch 11 und 12, **dadurch gekennzeichnet, dass** die Kontrolle der jeweiligen Mengen der abgelösten Tintenpartikel und der anhaftenden Tintenpartikel an eine Kontrolle des Weißgehalts im Rahmen der Verfeinerung der Regelung der Schritte des Ablösens, der Fragmentierung der Tmte, des Bleichens und/oder Entfarbens der Suspension gekoppelt ist.

## Claims

1. A device for continuously measuring the quantity of ink particles in suspension in a paper pulp coming from recycled paper, which particles are in detached form, that is to say individualized within the pulp, and in attached form, that is to say bonded to papermaking fibers, comprising :
• a separating and washing assembly (30 - 33) for separating the ink particles into respectively attached and detached fractions, said assembly being continuously fed with paper pulp via a suitable pipe (41), and comprising :
- a cylmdrical microperforated screen (30) on the inside of which the paper pulp to be analyzed is continuously conveyed via a pipe (41) ;
- a declogging and screening unit (32), intended to create pressure pulses and to force the paper pulp onto the internal surface of the screen (30) ;
- collection and outlet channels, respectively (10) for the undiluted detached ink particles, (39) for the diluted fine elements and (43) for the washed papermaking fibers with attached ink particles ;
• a measuring and processing assembly comprising :
- two channels or ducts (10, 11), coming from said separating assembly and conveying said fractions of ink particles to a measurement zone, each channel or duct having a transparent window in this zone ;
- a measurement and capture unit comprising :
◆ a unit (20) for illuminating said windows ;
◆ two cameras (12, 13) located opposite each of said windows, synchronized with the illumination unit (20) and intended to capture, at regular intervals, an image of the windows ;
- means (23) for digitizing the images thus captured and for processing them, these means being intended to determine the size and quantity of said ink particles;
- a unit for outputting the data determined in this way, especially to a sensory reproduction unit (24), or in the form of analog or digital signals intended to actuate one or more defined units.

2. The device as claimed in claim 1, wherein :
• the declogging and screening unit (32) is constituted by a rotor, the axis of rotation of which being collinear with the axis of the screen ;
• the collection and outlet channel (43) for the washed papermaking fibers with attached ink particles is located within the internal volume defined by the screen (30).

3. The device as claimed in claim 2, wherein the rotor (32) upstream of the separating and washing assembly, defines a deaeration chamber (44), in which the pipe (41) for conveying the paper pulp to be analyzed terminates, said deaeration chamber communicating with the outside via a vent (45) for removing the air bubbles contained in said pulp.

4. The device as claimed in either of claims 2 and 3, wherein the rotor is provided on its external peripheral surface with radial disks (37) in the region of which pipes conveying washing water terminate, said disks being equipped with openings for controlling the flow of the paper pulp upstream of the screen (30).

5. The device as claimed in either of claims 2 and 3, wherein the rotor is provided on its periphery with a spiraled screw in the region of which ducts conveying the washing water terminate.

6. The device as claimed in one of claims 2 to 5, wherein the rotor (30) is provided with declogging cylinders (40), these being provided near its periphery and extending parallel to the axis of rotation of the rotor.

7. The device as claimed in one of claims 2 to 6, wherein the screen (30) includes, downstream, a stationary spiraled channel intended to carry out countercurrent washing of the fibers.

8. A device for continuously measuring the quantity of ink particles in suspension in a paper pulp coming from recycled paper, which particles are in detached form, that is to say individualized within the pulp, and in attached form, that is to say bonded to papermaking fibers, comprising :
• a separating and washing assembly (30 - 33) for separating the ink particles into respectively attached and detached fractions, said assembly being continuously fed with paper pulp via a suitable pipe (41), and comprising :
- a flat microperforated screen (50) at the level of which the paper pulp to be analyzed is continuously conveyed via a pipe (53) ;
- a declogging and screening unit (51), intended to create pressure pulses and to force the paper pulp onto the surface of the screen (50),
- collection and outlet channels, respectively (57) for the undiluted detached ink particles, (59) for the diluted fine elements and (68) for the washed papermaking fibers with attached ink particles ;
• a measuring and processing assembly comprising :
- two channels or ducts (10, 11), coming from said separating assembly and conveying said fractions of ink particles to a measurement zone, each channel or duct having a transparent window in this zone ;
- a measurement and capture unit comprising :
◆ a unit (20) for illuminating said windows ;
◆ two cameras (12, 13) located opposite each of said windows, synchronized with the illumination unit (20) and intended to capture, at regular intervals, an image of the windows ;
- means (23) for digitizing the images thus captured and for processing them, these means being intended to determine the size and quantity of said ink particles ;
- a unit for outputting the data determined in this way, especially to a sensory reproduction unit (24), or in the form of analog or digital signals intended to actuate one or more defined units.

9. The device as claimed in claim 8, wherein the declogging and screening unit (51) consists of a rotor having a spiraled channel, which is made to rotate and the axis of rotation of which is coincident with the center of the screen (50), the rotation of said rotor pumping the paper pulp upstream of the screen and defining a chamber for deaerating said pulp in its central zone.

10. The device as claimed in claim 8, wherein the screen (50) includes, downstream, a stationary spiraled channel intended to carry out countercurrent washing of the fibers.

11. A process for regulating a line for deinking a paper pulp coming from recycled paper, which consists in continuously measuring the quantity of suspended ink particles that are individualized within the pulp, and the quantity of suspended ink particles that are bonded to other more voluminous particles, and especially to the papermaking fibers, by means of a device according to any one of claims 1 and 8, and then, depending on the measurements thus made, in acting on the various operating parameters of the deinking line, especially during the pulping and hot slushing steps, and during the washing and flotation steps.

12. The process for regulating a line for deinking a paper pulp coming from recycled paper as claimed in claim 11, wherein the measurement of the attached and detached ink particles is performed at the beginning of the deinking line and/or after a first deinking step, that is to say after flotation and/or washing, and/or after a second deinking step.

13. The process for regulating a line for deinking a paper pulp coming from recycled paper as claimed in either of claims 11 and 12, wherein the monitoring of the respective quantities of detached ink particles and attached ink particles is coupled with monitoring of the reflectance within the context of refining the regulation of the ink-detachment and ink-fragmentation steps and the pulp-bleaching and/or pulp-decoloring steps.
